(19) Europäisches Patentamt
European Patent Office
Office européen des brevets

(11) Publication number: **0 194 545 B1**

(12) # EUROPEAN PATENT SPECIFICATION

(45) Date of publication of the patent specification: **06.12.89**

(51) Int. Cl.⁴: **C07H 21/00** // C07H19/073, C07H19/10

(21) Application number: **86102766.2**

(22) Date of filing: **03.03.86**

(54) **Labelling of oligonucleotides.**

(30) Priority: **15.03.85 US 712481**
**18.12.85 US 808871**

(43) Date of publication of application:
**17.09.86 Bulletin 86/38**

(45) Publication of the grant of the patent:
**06.12.89 Bulletin 89/49**

(84) Designated Contracting States:
**AT BE CH DE FR GB IT LI LU NL SE**

(56) References cited:
**EP-A- 0 182 168**
**EP-A- 0 184 056**
**EP-A- 0 200 362**

**GENE, vol. 33, 1985, pages 191-196, Elsevier Science Publishers, New York, US; K.M. LANG et al.: "Cloning specific complete polyadenylylated 3'-terminal cDNA segments"**

(73) Proprietor: **MOLECULAR DIAGNOSTICS, INC., 400 Morgan Lane, West Haven, CT 06516(US)**

(72) Inventor: **Dattagupta, Nanibhushan, Dr., 470 Prospect Street, New Haven CT 06511(US)**
Inventor: **Knowles, William, Dr., 101 Sleeping Giant Dr., Hamden, CT(US)**

(74) Representative: **Jesse, Ralf-Rüdiger, Dr. et al, Bayer AG Konzernverwaltung RP Patentabteilung, D-5090 Leverkusen 1 Bayerwerk(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

BACKGROUND OF THE INVENTION

The present invention relates to methods of making an oligonucleotide. The present invention also concerns methods of making oligonucleotide probes.

It has been shown by Studencki and Wallace (DNA, 3, 7 (1984)) that an oligonucleotide can be labeled with radioactive $^{32}P$ using an enzyme and a combination of primer and template. The method is efficient and can produce highly labeled probe. The method has a serious disadvantage because the template and the products have identical electrophoretic mobility unless one is phosphorylated at the 5' end, or the template or the primer is tritylated at one end. This requires that one of the strands be labeled with $^{32}P$ using terminal labeling enzyme, or tritylated and 100% of these molecules should carry the modification.

The modification by phosphorylation is not efficient enough to produce 100% 5' end phosphorylated nucleic acids. Moreover, depending on the sequence, the mobility difference between phosphorylated template extended primer and the other strand may not be enough to separate them. 5'-end tritylated residues have the disadvantage of lability in aqueous solutions. The present invention is a method of separation of primer extended oligonucleotide probes from the templates by modification of one of the participating oligonucleotides. The modification is such that it is stable, hydrophobic/hydrophilic and changes the electrophoretic mobility and elution property on a reverse phase column and if desired reusable. It is desirable that the template be modified to carry the separability property. The synthesized labeled molecules should remain unmodified other than the modifications introduced by an enzyme reaction used in the synthesis.

As for example, if the template is modified at the 3' end with hexylamino ATP, the mobility of the template during gel electrophoresis will be slower than the synthesized product. Similar modifications will also give rise to separability during chromatography under high pressure on a reverse phase system.

SUMMARY OF THE INVENTION

The present invention concerns a method of making an oligonucleotide which comprises hybridizing (a) a shorter fragment of the desired oligonucleotide with (b) a nucleic acid fragment longer than (a) and complementary to the desired oligonucleotide, one of (a) and (b) carrying an organic radical which differentiates its physical properties relative to the other of (a) and (b), contacting the hybridized material with an enzyme and nucleoside triphosphates, whereby the shorter fragment grows in one direction until it is substantially coterminus with the complementary nucleic acid fragment, denaturing the hybridized material and separating lengthened (a) from (b); with the proviso that the organic radical is not a solid substrate. This proviso concerns the subject-matter of our application EP-A 184 056 which is prior art for the purposes of Art. 54(3) only. Such separation of the labelled material can be carried out by high performance liquid chromatography (HPLC) or by gel electrophoresis.

In the above described method, the organic radical can be a reactive site and separation can be effected by contact with a medium which reacts with the reactive site.

In the above described method, the nucleoside triphosphates can include a label. The label can be a radioactive element, e.g., radioactive phosphorus.

In the structures herein, the various symbols stand for the following nucleotide resides:

A = adenine nucleotide residue
C = cytosine nucleotide residue
G = guanosine nucleotide residue
T = thynidine nucleotide residue
U = uracile nucleotide residue

DETAILED DESCRIPTION OF THE INVENTION

The invention can be further described as follows:

Method 1

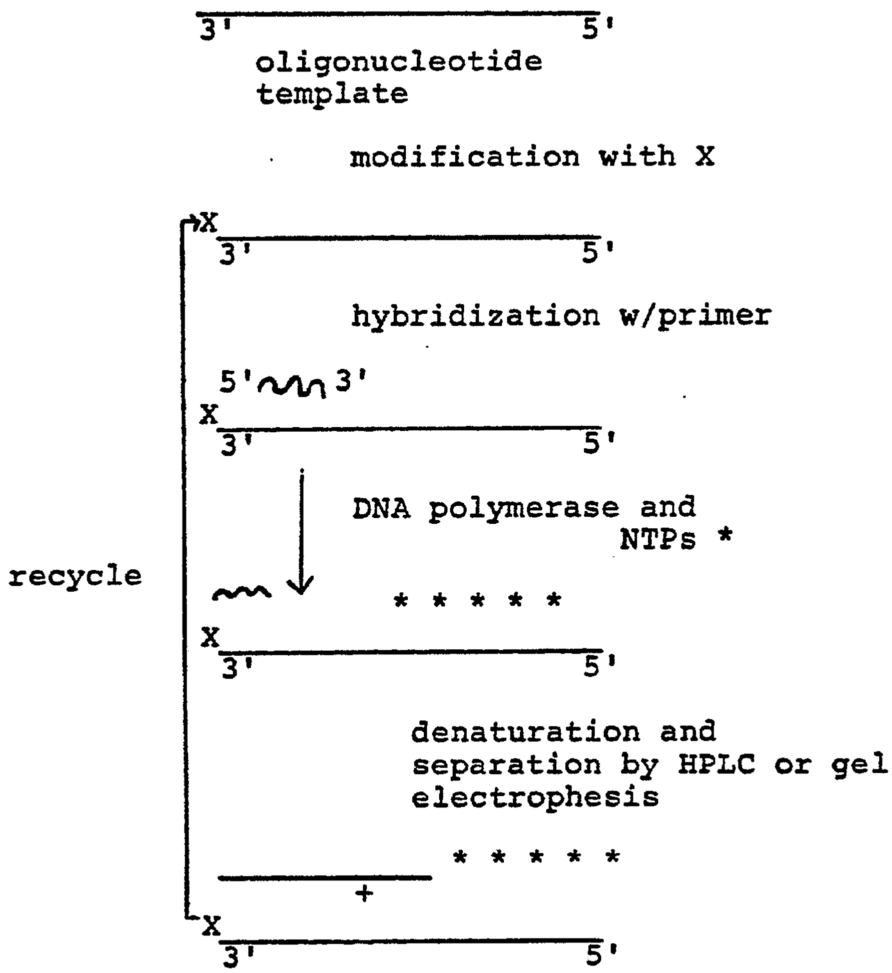


Instead of modifying the template, if a modified nucleoside triphosphate is used labeled strand can also be separated.

In order to maintain the fidelity of the process it is desirable that the modification of the template be done at the 3' or 5'end. If a primer is modified its 5' end is the desirable site of alteration. The modification is carried out via

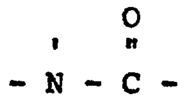,

$-S-S-$,

$-S-C-$,

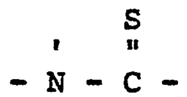,

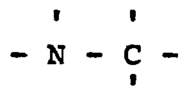,

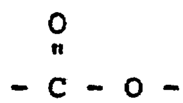,

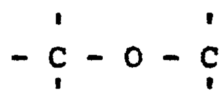, or

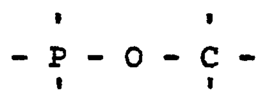

linkages using known reactions and modified nucleic acid residues at the specific site. The modifier can be ionic, nonionic, hydrophobic or hydrophilic. The modification can be before or after the labeling reactions. If the modification is carried out after, the labeling reagent should be reactive enough to efficiently modify all the desired residues.

As for example, an oligonucleotide with an amine residue (as in 8-hexylamino A or 5-allylamino U containing oligonucleotide) can be reacted with

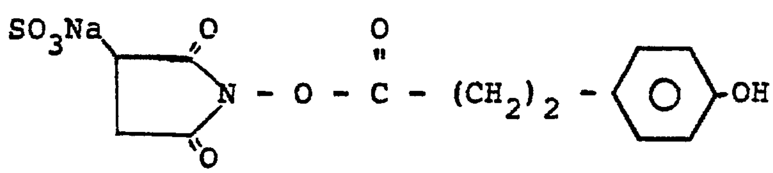

or similar reagents to form

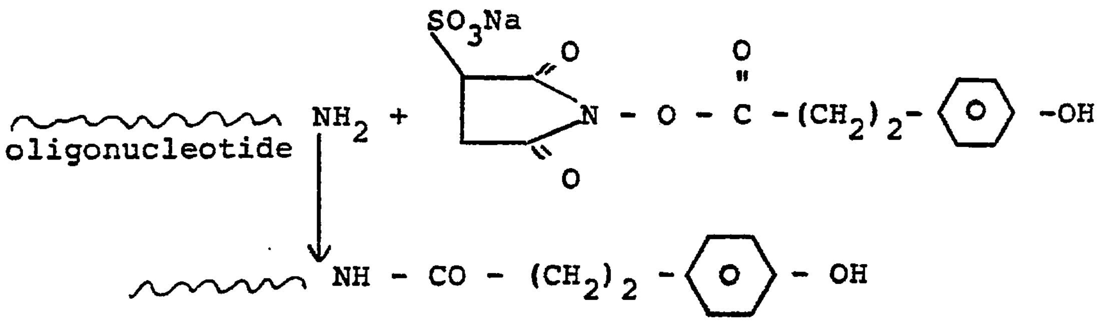


an oligonucleotide with hydrophobic

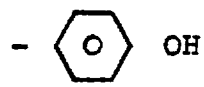

residue. A few other typical reactions are described below.

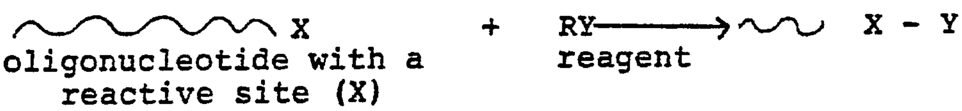


| X | RY | X-Y |
| --- | --- | --- |
| $NH_2$ | 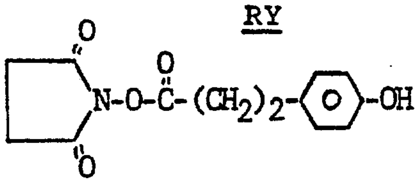 | 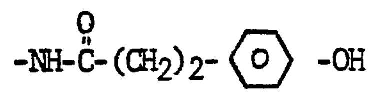 |
| | 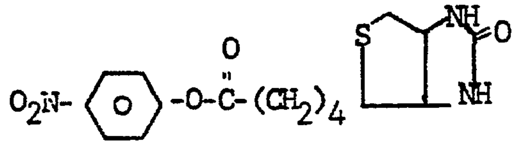 | 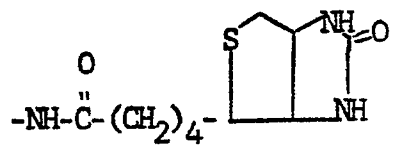 |

| | | |
|---|---|---|
| $\sim NH_2$ | isothiocyanate or isocyanate | $\sim NH-C(=S)-NH-$ |
| | Epoxide | $\sim NH-CH_2-CH(OH)-$ |
| $\sim SH$ | 2,4 dinitrochlorobenzene | 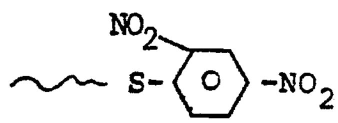 |
| | Iodoacetamide | $\sim S-CH_2-CONH_2$ |
| | Dansylhydrazine | $\sim S-CH_2-CH_2-NH$-dansyl |
| | SH- containing reagent | $\sim$ S-S-Reagent |
| $\sim COOH$ | Alcohol | Ester |
| $\sim OH$ | Epoxide | ether linkage |
| $\sim P(=O)(OH)-OH$ | Catalytic esterification (Y) | $\sim P(=O)(OH)-OY$ |

Any type of label can be introduced by using corresponding nucleoside substrates, for example, with biotinylated UTP, a biotin label and $^{32}P$ labelled NTP can be introduced.

The invention will now be described with reference to the following non-limiting examples.

Example 1: Synthesis of a modified oligonucleotidetemplate and modification with a hydrophobic residue

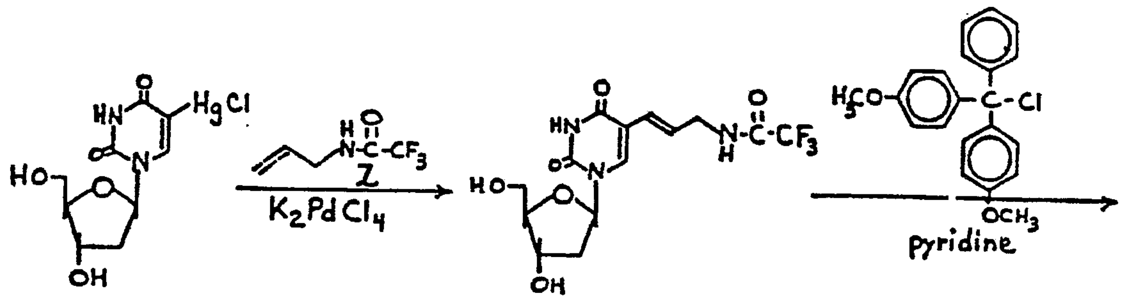


5 8

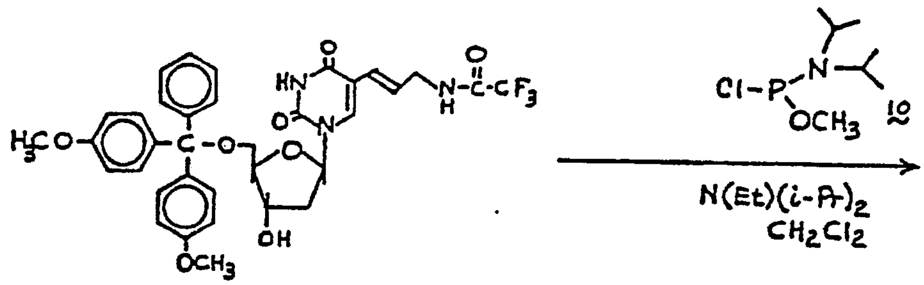


9

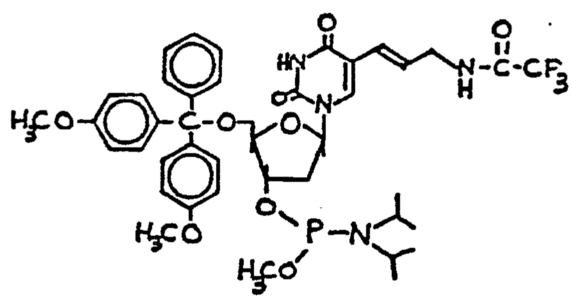

1

<u>Scheme for the synthesis of 1 to be added to 19A' at the 5' end.</u>

5-Chloromercuri-2'-deoxyuridine (compound 5), prepared according to the method of Bergstrom and Ruth (D.E. Bergstrom and J.L.Ruth, <u>J. Carbohydrates, Nucleosides and Nucleotides</u> 4 (5), 257 (1977)) was treated with 3-trifluoroacetamido-1-propene (compound 7) (M. Pailer and W.J. Hübsch, <u>Monatshefte für Chemie</u> 97 (6), 99 (1966)) and $K_2PdCl_4$ in methanol to give 5-trifluoroacetamidoallyl-2'-deoxyuridine (compound 8) in 22% yield after 2 chromatographies and a crystallization from methanol. Reaction of compound 8 with 4,4-dimethoxytrityl chloride in pyridine afforded compound 3 in 85% yield after flash chromatography (W.C. Still, M. Kahn and A. Mitra, <u>J. Org. Chem.</u> 43, 2923 (1978)), which was subsequently treated with N,N-diisopropylaminomethoxy chlorophosphine (L.J. McBride and M.H. Caruthers, <u>Tet. Letters</u> 24 (3), 245 (1983)) (compound 10) to give compound 1 as a white solid after precipitation from pentane.

A 19-unit oligonucleotide (I) was prepared using a DNA
Hβ19A': 3'-GA-GGA-CTC-CTC-TTC-AGA-CG-5'
(I)
synthesizer; three separate 1 μmole batches of each oligonucleotide were made and each was attached to a solid support and fully protected. The dimethoxytrityl protecting group was removed from the 5'-terminus and compound 1 was attached to the 19-unit chain without the DNA synthesizer, but using the same reagents and conditions the machine typically employs.

The result of this process is an oligonucleotide with a 5'-aminoallyl-5'-(4,4'-dimethoxytrityl)-2'-deoxyuridine unit at the C-5' end (II).

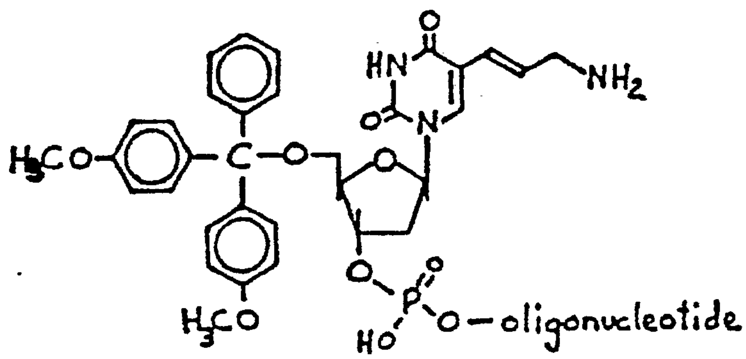

(II)

When portions of each of the modified oligonucleotides were treated with 3% trichloroacetic acid in methylene chloride, the distinctive red-orange color of liberated dimethoxytrityl cation was observed, indicating that the 5-allylamino-2'-deoxyuridine moiety had been attached. The polynucleotide were lastly de-tritylated with brief exposure to 3% trichloroacetic acid then purified by polyacrylamide gel electrophoresis. This demonstrates the utility of compound 1 as a synthon for introducing a 5-aminoallyl-2'-deoxyuridine unit at the C-5' terminus of an oligonucleotide; incorporation of this unit at any position in the oligonucleotide should be possible using the same methodology.

The process of attaching an analogue of a Bolton and Hunter (A.E. Bolton and W.M. Hunter, Biochem. J. 133, 529 (1973)) reagent is described in Scheme 2). Reaction of the amino-functionalized polynucleotide compound 11 with the N-hydroxysuccinimide ester of 3-(4'-hydroxyphenyl) propionic acid (compound 12) will yield the probe polynucleotide compound 13.

The polynucleotide H 19A' is 19 unit polynucleotides corresponding to a portion of human DNA which codes for the polypeptide hemoglobin, specifically that region of the DNA wherein lies the mutation which manifests itself in the formation of sickle-cell hemoglobin and the genetic disorder known as sickle cell anemia.

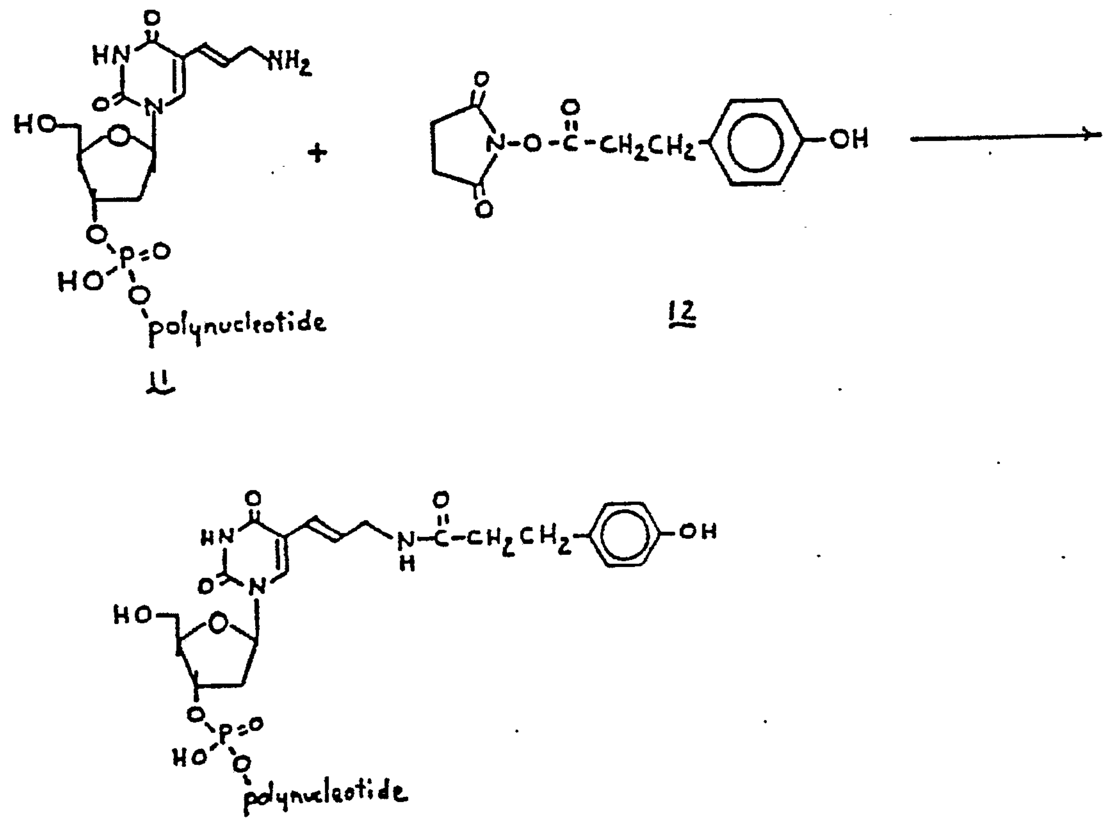

11

12

Scheme 2

Infrared (IR) spectra were obtained with a Perkin-Elmer Model 710B or 237 infrared spectrophotometer as solutions in $CHCl_3$ unless otherwise noted; the 1602 $cm^{-1}$ band of polystyrene film was used as an external calibration standard. Signals are reported as $cm^{-1}$.

Proton magnetic resonance ($^1H$ NMR) spectra were obtained at 89.55 MHz using a Varian T-60 spectrometer; spectra were obtained in $CDCl_3$ solution unless otherwise noted. Chemical shifts are reported in parts per million downfield from the internal standard tetramethylsilane, unless otherwise noted.

Carbon-13 magnetic resonance ($^{13}C$ NMR) spectra were obtained at 22.5 MHz using a JEOL FX90Q spectrometer with Fourier transform and with full proton broad-band noise decoupling; spectra were obtained in $CDCl_3$ solution unless otherwise noted. Carbon shifts are reported in parts per million downfield from the internal standard tetramethylsilane, unless otherwise noted.

Phosphorus-31 magnetic resonance ($^{31}PNMR$) spectra were obtained at 36.21 MHz using a JEOL FX90Q spectrometer; spectra were obtained in $CDCl_3$ solution unless otherwise noted. Phosphorus shifts are reported in parts per million downfield from an external aqueous 15% $H_3PO_4$ standard.

Optical rotations were obtained on a Perkin-Elmer Model 141 Polarimeter.

Organic reagents were obtained from Aldrich Chemical Company and were used without purification, unless otherwise noted. Inorganic reagents were ACS reagent grade from Fisher Scientific Company or other major vendor. Reaction solvents were ACS reagent grade. Reagents used in oligonucleotide synthesis were obtained from Applied Biosystems, Inc. Brine refers to a saturated aqueous sodium chloride solution.

Thin layer chromatography (TLC) was performed using silica gel 60F-254 plates from E. Merck. Column chromatography was performed using E. Merck Silica Gel 60 (70-230 mesh). All melting points reported are uncorrected.

5-Trifluoroacetamidoallyl-2'-deoxyuridine (compound 8)

A suspension of 5-chloromercuri-2'-deoxyuridine (compound 5) (D.E. Bergstrom and J.L. Ruth, J.Carbohydrates, Nucleosides and Nucleotides 4(5), 257 (1977)) (5.56 g; 12 mmol) in high performance liquid chromatography (HPLC) grade methanol (120 ml) was maintained under an inert gas atmosphere at ambient temperature and treated with 3-trifluoroacetamido-1-propene (compound 7) (M. Pailer and W.J. Hübsch, Monatshefte für Chemie, 97(6), 99 (1966)) (7.33 g; 48 mmol; 4 eq) and $K_2PdCl_4$ (4.28 g; 1.1 eq). The reaction gradually became black and was allowed to stir for 22 hours. The mixture was

treated with $H_2S$ gas for several minutes then filtered through Celite, rinsed with MeOH and evaporated to dryness under reduced pressure from a 80°C bath to give a crude semi-solid residue (7.0 g). The residue was chromatographed on a silica gel column developed with $CH_2Cl_2$ / MeOH (5:1). The band which stained a blue color with modified p-anisaldehyde reagent (Thin Layer Chromatography by Egon Stahl, 2nd ed. Springer-Verlag, New York 1969, page 857) and had an Rf = 0.51 ($CH_3CN$ / MeOH 3:1) was collected and evaporated to dryness in vacuo to give a colorless foam. The product was crystallized from a minimum of methanol, filtered, washed with cold $CHCl_3$ / MeOH (3:1) and vacuum dried. The mother liquor was worked for a second crop-total yield 1.01 g (22%). A recrystallization from MeOH afforded the title compound (8) as analytically pure tiny white needles with mp = 183-4°C after drying in vacuo (<1.0 torr) at 64°C overnight. IR (KBr) $cm^{-1}$ 3420, 3260 , 1718, 1683 (br), 1560, 1478, 1283, 1190, 1102, 1061, 980, 788, 763, 737; 'HNMR (DMSO-$d^6$) (Ref.-DMSO-$d^6$) 2.13 (d of d, J = 6 Hz, 2H), 3.59 (br s, 2H), 3.70-3.97 (m, 3H), 4.25 (br s, 1H), 5.06 (br m, 1H), 5.20 (br m, 1H), 6.05-6.65 (m 4H), 8.01 (s, 1H), 9.60 (br s 1H); $^{13}C$ NMR (DMSO-$d^6$) (Ref. DMSO-$d^6$)ppm 162.05, 155.29, 149.50, 138.05, 124.33, 124.14, 109.96, 87.53, 84.47, 70.23, 61.12, 39.93; $[\alpha]_D = +8.01°$ (c = 0.87, MeOH).

Anal. Calcd. for $C_{14}H_{16}N_3O_6F_3$ : C, 44.33; H, 4.25; N, 11.08
Found : C, 44.19; H, 4.10; N, 10.93

5-Trifluoroacetamidoallyl-5'-0-(4,4'-dimethoxytrityl)-2'-deoxyuridine (compound 9)

A solution of compound 8 (0.60 g; 1.58 mmol) in anhydrous pyridine (8 ml) was maintained under an inert gas atmosphere and treated at ambient temperature with 4,4'-dimethoxytrityl chloride (0.67 g; 1.25 eq). After stirring for 18 hr the reaction was poured into ice water (70 ml) with vigorous shaking. On standing 1/3 hr at 0° a gummy solid separates leaving a nearly clear solution which was decanted. The solid was washed once with $H_2O$ (5 ml) then taken up in $CH_2Cl_2$ (10 ml), washed once with brine (5 ml) then the $CH_2Cl_2$ solution was dried over $K_2CO_3$, filtered and evaporated to dryness in vacuo to give a brownish foam. The crude product was purified by flash chromatography (W.C. Still, M. Kahn and A. Mitra, J. Org. Chem. 43, 2923 (1978)) on a column of silica gel (Merck, Grade 60, 230-400 mesh, 60 A)(75 g) developed with 4.0% MeOH in $CHCl_3$ solvent (1.0 liter). Fractions of ca. 20 ml each were collected in tubes containing pyridine (10 µl) to inhibit deprotection of the 5'-hydroxyl. Fractions containing the major product band (RF = 0.29; MeOH / $CHCl_3$ 7:93) were combined, filtered and evaporated to dryness in vacuo to give compound 9 (0.91 g; 85%) as a slightly yellowish foam. A fraction from the center of the elution band was freed of solvent, taken up in EtoAc, treated with Norit 211, filtered through Celite and evaporated to dryness under high vacuum (<1.0 torr) at 64°C overnight to afford the analytical sample as a colorless foam with mp = 105-110°C (dec.). IR ($CHCl_3$) $cm^{-1}$ 3370, 2920, 1715, 1695, 1618, 1515, 1470, 1260, 1182, 1045, 842; 'H NMR ($CDCl_3$) δ2.38 (br m, 2H), 3.25-3.75 (m, 5H), 3.75 (s, 6H), 4.10 (br m IH), 4.60 (br s, 1H), 5.39 (d, J = 16 Hz, 1H), 6.10-6.55 (m, 2H), 6.70-6.95 (m, 5H), 7.15-7.45 (m, 10H), 7.84 (s, 1H); $^{13}C$ NMR ($CDCl_3$) (Ref. $CDCl_3$) ppm 162.31, 158.74, 157.70, 156.01, 149.70, 144.04, 137.88, 135.65, 135.52, 130.12, 128.11, 127.26, 125.05, 113.48, 111.33, 86.94, 86.68, 85.25, 72.18, 63.60, 55.34, 42.66, 41.42.

Anal. Calcd. for $C_{35}H_{34}N_3O_8F_3$ : C, 61.67; H, 5.03; N, 6.16
Found : C, 61.47; H, 5.19; N, 5.95

5-Trifluoroacetamidoaminoallyl-5'-0-(4,4'-dimethoxy-trityl)-2'-deoxyuridine-3'-0-(N,N-diisopropylamino-methoxy phosphine) (Compound 1)

A solution of compound 9 (0.34 g; 0.5 mmol) in anhydrous $CH_2Cl_2$ (1.5 ml) maintained under an Argon atmosphere at ambient temperature was treated first with anhydrous diisopropylethylamine (0.35 ml; 0.259 g; 2 mmol; 4 eq) then dropwize, over 1 minute, with N,N-diisopropylaminomethoxychlorophosphine (L.J. McBride and M.H. Caruthers, Tet. Letters 24 (3), 245 (1983)) (compound 10) (0.19 ml; ca. 0.2 g; 2.2 eq). The resultant colorless solution is stirred for 20 minutes then transferred with EtOAc (20 ml) (EtOAc was previously washed with saturated aq $NaHCO_3$ then brine) to a separatory funnel, washed four times with brine (35 ml each), dried over $N_2SO_4$, filtered and evaporated to dryness in vacuo to give a colorless glass (0.51 g). This crude product was taken up in anhydrous benzene (2 ml) and precipitated into rapidly stirred anhydrous pentane (60 ml) at -78°C under an Argon atmosphere. The resulting suspension was filtered, washed with -78°C pentane and vacuum dried at < 1 torr over KOH overnight to obtain the title compound 1 (0.38 g; 93%) as a white amorphous powder. IR ($CHCl_3$) $cm^{-1}$ 2965, 1722, 1698, 1618, 1518, 1470, 1262, 1185, 1045, 988, 842; 'H NMR ($CD_2Cl_2$) δ0.95-1.30 (m, 12H), 2.20-2.60 (m, 2H), 3.24 and 3.37 (d of d, J = 13 hz, 3H) (P-O-$CH_3$), 3.20-3.80 (m, 6H), 3.75 (s, 6H), 4.17 (br m, 1H), 4.68 (v br m, 1H), 5,42 (d, J = 16 Hz, 1H), 6.15-6.55 (m, 3H), 6.75-6.95 (m, 4H), 7.20-7.50 (m, 10H), 7.79 (s, 1H); $^{13}C$ NMR ($CD_2Cl_2$) (Ref. $CD_2Cl_2$) ppm 162.40, 159.21, 157.78, 149.78, 144.71, 138.34, 136.00, 130.53, 128.71, 128.45, 127.54, 125.66, 125.27, 113.82, 111.48, 87.23, 86.31, 85.60, 55.75, 43.78, 43.20, 42.94, 24.99, 24.60; $^{31}P$NMR ($CD_2Cl_2$) ppm 149.30, 148.87, 14.11 (ca. 12% impurity), 8.18 (ca. 4% impurity).

Attachment of Compound 1 to Oligonucleotides

The 19-unit oligonucleotides were synthesized using an Applied Biosystems Model 380A DNA Synthesizer on control pore glass solid support. Immediately prior to attaching compound 1 to the 5' end of the oligomer, the 5'-0-(4,4'-dimethoxytrityl) protecting group was cleaved on the machine with 3% $CCl_3CO_2H$ in $CH_2Cl_2$ for 90 seconds. The support-bound 5'-deprotected oligomer was washed with $CH_3CN$ and dried in an Argon stream. Subsequent steps were performed without the machine, but using the same chemistry;

(1) The support-bound oligomer was removed from the container (column) used for automated synthesis and transferred to a dry septum-cap vial under an Argon atmosphere.
(2) The bound oligomer was treated with a 20-30 fold excess of 0.5 M 1H-Tetrazole in anhydrous $CH_3CN$ followed immediately with a similar excess of compound 1 in $CH_3CN$. Incubate 30 minutes with gentle agitation.
(3) Pipette off reagents and wash bound oligomer with 3 portions of $CH_3CN$.
(4) Treat with an excess of $I_2$-$H_2O$-Lutidine-THF (0.1 M : 1:10:40) and agitate for 15 minutes.
(5) Pipette off reagent and wash bound oligomer with 4 portions of $CH_3CN$.
(6) Treat with an excess of Thiophenol-triethylamine-dioxane for 60 minutes.
(7) Pipette off reagent and wash the bound oligomer with 4 portions of MeOH.
(8) Treat with concentrated aq. $NH_4OH$ for 2 hours at ambient temperature (removes protected oligonucleotide from the support).
(9) Add more concentrated aqueous $NH_4OH$ and heat at 50°C overnight (removes all protecting groups except the dimethoxytrityl).
(10) Filter off the support and evaporate the filtrate to dryness to get crude oligonucleotide.

This was repeated for all batches of support-bound oligonucleotide. Treatment of a portion of each on a silica gel thin layer chromatography (TLC) plate with 3% $CCl_3CO_2H$ in $CH_2Cl_2$ produced the orange-red color of dimethoxytrityl cation indicating the successful incorporation of compound 1 into the oligonucleotides.

One batch of the modified Hβ19A' oligonucleotide was detritylated with 3% $CCl_3CO_2H$ in $CH_2Cl_2$ then purified by electrophoresis on a 20% polyacrylamide gel under denaturing conditions.

Example 2: The reaction of a specific oligonucleotide with sulfosuccinimidyl hydroxy phenyl propionate

Two micrograms of 19A' amine product of Example 1 is dissolved in 20 microliter of 10 mM borate buffer pH 8.16. To this 5 microliter of a freshly prepared solution of sulfosuccinimidyl 3-(4-hydroxyphenyl) propionate (SHPP) purchased from Pierce Chemical Co., Rockford, Illinois, U.S.A. is added. The concentration of SHPP is 5 mg per ml in water. The reaction is allowed to proceed at room temperature for 30 minutes, then it is kept in the freezer before HPLC separation is done.

After the reaction, the extent of the reaction is monitored by running a 20% polyacrylamide gel in conventional manner.

Example 3: Separation of the SHPP reacted 19A' from the reaction mixture containing hydrolyzed SHPP and oligonucleotides

A HPLC run has been done on a Brownlee RP300 guard column coupled to a Synchrome RP-P 4.1 x 10 cm column at ambient temperature. A gradient of (a) .1 M triethylammonium acetate pH 7 with (b) 0.1 M triethylammonium acetate pH 7.0, 50% acetonitrile at a flow rate of 1 ml/minute. The UV detector at 254mm is used to monitor the elution of oligonucleotide. In order to find out the location of the product, a blank run has been done with all of the reactants in the starting mixture without the oligonucleotide. The new peak appeared after adding the oligonucleotide is taken to be the peak corresponding to the reaction product. After the product is separated and collected in a fraction collector, the product is analyzed by gel electrophoresis and from the next run an analytical determination of the proper peak is not necessary. The yield and recovery of the product from the columns is between 80% to 90%.

Example 4: Primer extension reaction for labeling an oligonucleotide

The product of Examples 1 or 3 can be used as template and both are separable by HPLC (high pressure liquid chromatography) or gel electrophoresis. An example is provided with the product of Example 1. An identical procedure can be followed with the product of Example 3.

Reaction conditions have been discussed by Studencki and Wallace, DNA, 3, 7 (1984). The primer extension reaction mixture contained 25 ul total volume and is prepared by mixing to produce a final concentration of 1.6 uM template
(3' GAGGACTCCTCTTCAGACG U*–5')
(U* is 5-allylamino U)

and 5.0 μM primer
(5' CTCCTGAGGAG – 3'
and 5 uM each of dATP, dTTP, alpha-[$^{32}$P] dCTP and alpha-[$^{32}$P] dGTP, and 10 units of E. coli DNA polymerase I (Klenow fragment) purchased from New England Nuclear, Boston, Mass., U.S.A. The reaction is allowed to proceed for 18 hours at room temperature.

Example 5: Purification of the product by gel electrophoresis

When the product of Example 1 or 3 is used as templates, the electrophoretic mobility (on a 20% polyacrylamide gel) of the product is different than the template and the short primer. The reaction mixture (Example 4) is diluted to 40 μl with loading buffer containing 8 M urea, 0.05% xylene cyanol FF and 0.05 Bromophenol blue in 20 mM tris EDTA buffer (pH 7.5). The mixture 40 μl is loaded onto a 20% polyacrylamide slab gel (0.2 x 15.5 x 29.5 cm) which has been preelectrophoresed for 1 hour at 1000 V. The gel is run for 8 hours at 1000 V in 50 mM trisborate buffer pH 8.3. After the run, autoradiographic detection is conducted to locate the radioactive product band; it is excised and eluted from the gel using 1 mM EDTA solution as described by Studencki and Wallace (DNA, 3, 7 (1984).

Example 6: Purification of the product by high pressure liquid chromatography

Both the products of Examples 1 and 3 are more hydrophobic than their parent oligonucleotide. They can be separated from the labeled complementary stand on a reverse phase system using acetronitrile gradient. An identical device and reaction conditions as have been described in Example 3 are used to separate the labeled product from the template. In this method a part of the primer (unreacted) coelutes with the product. This can be purified on a gel filtration system.

Example 7: Hybridization of the product of Example 5 or 6 with DNA from the beta-globin gene

The specific oligonucleotide fragment chosen for the examples (5,6) is usable for the detection of sickle cell anemia. This particular sequence of oligonucleotide will hybridize with DNA extracted from normal blood and under certain conditions it will not hybridize to the DNA extracted from the blood of a person who has sickle cell anemia disease. The extraction of DNA from blood samples and the digestion with a restriction enzyme and other appropriate treatment of the hybridization process have been described by Studencki and Wallace (DNA, 3 (1984) 7)). The newly labeled material shows similar hybridization property as a $^{32}$P labeled probe as has been described by Studencki & Wallace (DNA, 3 (1984) 7)).

It will be understood that the specification and examples are illustrative but not limitative of the present invention and that other embodiments within the spirit and scope of the invention will suggest themselves to those skilled in the art.

**Claims**

1. A method of making an oligonucleotide which comprises hybridizing (a) a shorter fragment of the desired oligonucleotide with (b) a nucleic acid fragment longer than (a) and complementary to the desired oligonucleotide, one of (a) and (b) carrying an organic radical which differentiates its physical properties relative to the other of (a) and (b), contacting the hybridized material with an enzyme and nucleoside triphosphates whereby the shorter fragment grows in one direction until it is substantially coterminus with the complementary nucleic acid fragment, denaturing the hybridized material and separating lengthened (a) from (b); with the proviso that the organic radical is not a solid substrate.

2. The method according to claim 1, wherein the organic radical is a reactive site and separation is effected by contact with a medium which reacts with the reactive site.

3. The method according to any of claims 1 and 2, wherein (b) carries the organic radical.

4. The method according to any of claims 1 to 3, wherein the organic radical is a hydroxyphenyl radical, or a polyacrylic acid radical.

5. The method according to any of claims 1 to 4, wherein the nucleoside triphosphates include a label.

6. The method according to claim 5, wherein the label is a radioactive element.

**Revendications**

1. Procédé de production d'un oligonucléotide, qui consiste à hybrider (a) un fragment plus court de l'oligonucléotide désiré avec (b) un fragment d'acide nucléique plus long que le fragment (a) et complémentaire de l'oligonucléotide désiré, l'un des fragments (a) et (b) portant un radical organique qui différencie ses propriétés physiques de celles de l'autre des fragments (a) et (b), à mettre en contact la matière hybridée avec un enzyme et des nucléoside-triphosphates, ce qui provoque la croissance du fragment plus court dans une direction pratiquement jusqu'à sa coterminaison avec le fragment d'acide nucléique complémentaire, à dénaturer la matière hybridée et à séparer le fragment allongé (a) du fragment (b), sous réserve que le radical organique ne soit pas un substrat solide.

2. Procédé suivant la revendication 1, dans lequel le radical organique est un site réactif et la séparation est effectuée par mise en contact avec un milieu qui réagit avec le site réactif.

3. Procédé suivant l'une quelconque des revendications 1 et 2, dans lequel le fragment (b) porte le radical organique.

4. Procédé suivant l'une quelconque des revendications 1 à 3, dans lequel le radical organique est un radical hydroxyphényle ou un radical acide polyacrylique.

5. Procédé suivant l'une quelconque des revendications 1 à 4, dans lequel les nucléosidetriphosphates comprennent un marqueur.

6. Procédé suivant la revendication 5, dans lequel le marqueur est un élément radio-actif.

## Patentansprüche

1. Verfahren zur Herstellung eines Oligonucleotids, welches beinhaltet, daß man (a) ein kürzeres Fragment des gewünschten Oligonucleotids mit (b) einem Nucleinsäurefragment hybridisiert, das länger als (a) und komplementär zum gewünschten Oligonucleotid ist, wobei eines von (a) und (b) einen organischen Rest trägt, der dessen physikalische Eigenschaften bezüglich des anderen vom (a) und (b) unterschiedlich macht, daß man ferner das hybridisierte Material mit einem Enzym und Nucleotriphosphaten in Kontakt bringt, wobei das kürzere Fragment in einer Richtung wächst, bis es im wesentlichen mit dem komplementären Nucleinsäurefragment coterminiert ist, daß man das hybridisierte Material denaturiert und schließlich verlängertes (a) von (b) trennt, mit der Maßgabe, daß der organische Rest nicht ein festes Substrat ist.

2. Verfahren gemäß Anspruch 1, dadurch gekennzeichnet, daß der organische Rest eine reaktive Stelle darstellt und die Trennung durch Kontakt mit einem Medium bewirkt wird, welches mit der reaktiven Stelle reagiert.

3. Verfahren gemäß jedem der Ansprüche 1 und 2, dadurch gekennzeichnet, daß (b) den organischen Rest trägt.

4. Verfahren nach jedem der Ansprüche 1 bis 3, dadurch gekennzeichnet, daß der organische Rest ein Hydroxyphenylrest oder ein Polyacrylsäurerest ist.

5. Verfahren gemäß jedem der Ansprüche 1 bis 4, dadurch gekennzeichnet, daß die Nucleosidtriphosphate eine Markierung einschließen.

6. Verfahren gemäß Anspruch 5, dadurch gekennzeichnet, daß die Markierung ein radioaktives Element ist.